# EUROPEAN PATENT SPECIFICATION

(11) **EP 2 177 572 B1**
(45) Date of publication and mention of the grant of the patent: **22.05.2013**
(21) Application number: 08838726.1
(22) Date of filing: 26.09.2008
(51) Int. Cl.: C09B 23/14, C09B 23/00, B41M 5/26, C07D 209/08, C07D 209/60, G11B 7/246, G11B 7/247

(54) **INDOLIUM COMPOUND AND OPTICAL RECORDING MATERIAL USING THE COMPOUND**
INDOLVERBINDUNG UND OPTISCHES AUFZEICHNUNGSMATERIAL MIT DER VERBINDUNG
COMPOSE INDOLIUM ET MATERIAU D'ENREGISTREMENT OPTIQUE UTILISANT LE COMPOSE

(30) Priority: 15.10.2007 JP 2007267683
(43) Date of publication of application: 21.04.2010
(73) Proprietor: Adeka Corporation, Tokyo 116-8554 (JP)
(72) Inventor: YANO, Toru, Tokyo 116-8554 (JP); AOYAMA, Yohei, Tokyo 116-8554 (JP)
(74) Representative: Forstmeyer, Dietmar
(86) International application number: PCT/JP2008/067514
(87) International publication number: WO 2009/051000

(56) References cited:
- EP-A1- 1 772 282
- EP-A1- 1 826 244
- EP-A1- 1 852 420
- EP-A1- 2 003 172
- WO-A1-2007/114074
- JP-A- 2006 124 542
- JP-A- 2006 150 841
- JP-A- 2006 150 841
- SETH DALY ET AL: "Unprecedented C-2 arylation of indole with diazonium salts: Syntheses of 2,3-disubstituted indoles and their antimicrobial activity", BIOORGANIC & MEDICINAL CHEMISTRY LETTERS, vol. 21, no. 16, 1 August 2011 (2011-08-01), pages 4720-4723, XP55010550, ISSN: 0960-894X, DOI: 10.1016/j.bmcl.2011.06.081
- MUNAWAR HUSSAIN ET AL: "Synthesis of carbazoles and 1,2-dihydrocarbazoles by domino 'twofold Heck/6[pi]-electrocyclization' reactions of di-, tri- and tetrabromoindoles", TETRAHEDRON, vol. 67, no. 29, 1 July 2011 (2011-07-01), pages 5304-5318, XP55010551, ISSN: 0040-4020, DOI: 10.1016/j.tet.2011.05.014
- GANESAN GOBI RAJESHWARAN ET AL: "Synthetic Studies on Indolocarbazoles: Total Synthesis of Staurosporine Aglycon", ORGANIC LETTERS, vol. 13, no. 6, 18 March 2011 (2011-03-18) , pages 1418-1421, XP55010552, ISSN: 1523-7060, DOI: 10.1021/ol200094b

## Description

### TECHNICAL FIELD

This invention relates to a novel indolium compound used mainly in an optical recording material and an optical recording material containing the indolium compound. More particularly, it relates to an optical recording material used in an optical recording medium on which information can be written as an information pattern mostly with a laser beam, especially an optical recording medium capable of high-density optical writing and reading using a low energy laser having a wavelength in the visible to ultraviolet region.

### BACKGROUND ART

Optical recording media have widely spread generally because of their superiority, such as high recording capacity and non-contact write/read system. Recordable optical disks, such as WORMS, CD-Rs, and DVD±Rs, record information by irradiating a very small area of the optical recording layer thereof with a focused laser beam to change the properties of the irradiated area and reproduce the recorded information making use of the difference in reflected light quantity between the recorded and non-recorded areas.

Compounds having an intense absorption, particularly an absorption maximum (λₘₐₓ), in the range of from 550 to 620 nm are used as an optical recording material forming an optical recording layer of an optical recording medium, such as DVD-R.

There are many reports on indolium compounds having an indole ring for use as the optical recording material discussed above in view of their high sensitivity and possibility to respond to the increasing writing speed. For example, patent documents 1 to 6 listed below report styryl indolium compounds. Patent document 7 (see below) reports a low temperature decomposing cyanine compound having an indole ring and a benzyl group introduced into the 3-position of the indole ring. Patent document 8 (see below) discloses an indolium compound having a benzyl group introduced to the 3-position of the indole ring. Low temperature decomposing compounds readily form recorded portions (pits) in an optical recording layer and are considered suited for use in high-speed recording media. However, these materials are unsatisfactory in performance properties including heat resistance, light resistance, and recording characteristics.

Patent document 1: JP 11-34489A
Patent document 2: JP 11-170695A
Patent document 3: JP 2001-342366A
Patent document 4: JP 2002-206061A
Patent document 5: JP 2003-313447A
Patent document 6: JP 2003-321450A
Patent document 7: JP 2003-231359A
Patent document 8: JP 2006-150841A

### DISCLOSURE OF THE INVENTION

### Problem to be Solved by the Invention

An object of the invention is to provide an indolium compound having high heat and light resistance and exhibiting thermal decomposition behavior suited for use in high-speed optical recording and an optical recording material containing the indolium compound.

### Means for Solving the Problem

As a result of extensive investigations, the present inventors have found that an indolium compound having a specific cation structure exhibits good thermal decomposition behavior as an optical recording material and thus reached the present invention.

The above object of the invention is accomplished by the provision of an indolium compound represented by general formula (I):

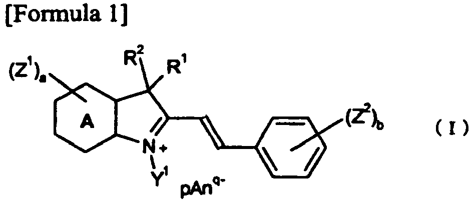

wherein ring A represents a benzene ring or a naphthalene ring; R¹ represents a group represented by general formula (II) or (III); R² represents an organic group having 1 to 30 carbon atoms or a group represented by general formula (II) or (III); Y¹ represents a hydrogen atom, an alkyl group having 1 to 10 carbon atoms, an aryl group having 6 to 20 carbon atoms, or an arylalkyl group having 7 to 20 carbon atoms; Z¹ represents an alkyl group having 1 to 8 carbon atoms optionally substituted with a halogen group and optionally interrupted by -O-, -CO-, -OCO-, or -COO-, a sulfonyl group having a hydrocarbon group having 1 to 8 carbon atoms, a sulfinyl group having a hydrocarbon group having 1 to 8 carbon atoms, an alkylamino group having an alkyl group containing 1 to 8 carbon atoms, a dialkylamino group having an alkyl group containing 1 to 8 carbon atoms, a group represented by general formula (III), a cyano group, a nitro group, a hydroxyl group, or a halogen group; Z² represents an alkyl group having 1 to 8 carbon atoms optionally substituted with a halogen group and optionally interrupted by -O-, -CO-, -OCO-, or -COO-, a sulfonyl group having a hydrocarbon group having 1 to 8 carbon atoms, a sulfinyl group having a hydrocarbon group having 1 to 8 carbon atoms, a group represented by general formula (III), a cyano group, a nitro group, a hydroxyl group, or a halogen group; a plurality of Z² substituents may be joined to form a ring structure; a represents an integer of 0 to 6; b represents an integer of 0 to 5; An^{q-} represents a q-valent anion; q represents 1 or 2; and p represents a number necessary to neutralize an electric charge,

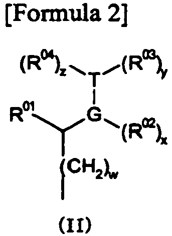

wherein the bond between G and T is a double bond, a conjugated double bond, or a triple bond; G represents a carbon atom; T represents a carbon atom, an oxygen atom, or a nitrogen atom; x, y, and z each represent 0 or 1, provided that, when T is oxygen, y=z=0, and, when T is nitrogen, y+z=0 or 1; w represents an integer of 0 to 4; R⁰¹, R⁰², R⁰³, and R⁰⁴ each independently represent a hydrogen atom, a hydroxyl group, a nitro group, a cyano group, a halogen atom, an alkyl group having 1 to 4 carbon atoms optionally substituted with a halogen atom, or an alkoxy group having 1 to 4 carbon atoms optionally substituted with a halogen atom; R⁰¹ and R⁰⁴ may be joined to form a cycloalkene ring or a heterocyclic ring,

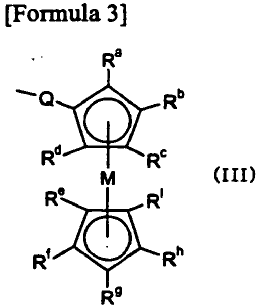

wherein R^{a}, R^{b}, R^{c}, R^{d}, R^{c}, R^{f} R^{g}, R^{h}, and Rⁱ each independently represent a hydrogen atom or an alkyl group having 1 to 4 carbon atoms a methylene moiety of which may be displaced by -O- or -CO-; Q represents a single bond or an optionally substituted alkylene group having 1 to 8 carbon atoms a methylene moiety of which may be displaced by -O-, -S-, -CO-, -COO-, -OCO-, -SO₂-, -NH-, -CONH-, -NHCO-, -N=CH-, or -CH=CH-; and M represents Fe, Co, Ni, Ti, Cu, Zn, Zr, Cr, Mo, Os, Mn, Ru, Sn, Pd, Rh, Pt, or Ir.

In a preferred embodiment of the invention, the object of the invention is accomplished by the provision of the indolium compound of general formula (I) which is represented by general formula (IV):

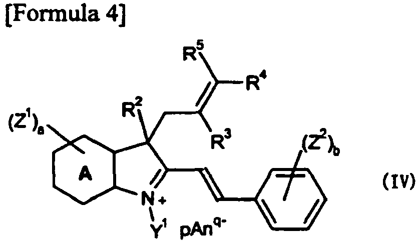

wherein ring A, R², Y¹, Z¹, Z², a, b, An^{q-}, p, and q are as defined for general formula (I); and R³, R⁴, and R⁵ each independently represent a halogen atom, a hydroxyl group, a nitro group, a cyano group, an alkyl group having 1 to 4 carbon atoms optionally substituted with a halogen atom, or an alkoxy group having 1 to 4 carbon atoms optionally substituted with a halogen atom.

The object of the invention is also accomplished by providing an optical recording material containing at least one indolium compound of the invention, which is used to form an optical recording layer on a substrate to provide an optical recording medium.

The object of the invention is also accomplished by providing an optical recording medium including a substrate and an optical recording layer on the substrate, the optical recording layer being formed of the optical recording material of the invention.

### BEST MODE FOR CARRYING OUT THE INVENTION

The indolium compound of the invention and the optical recording material containing the indolium compound will be described in detail with their preferred embodiments.
Indolium compounds represented by general formulae (I) and (IV) will be described first.

In general formulae (I) and (IV), the alkyl group having 1 to 8 carbon atoms that is optionally substituted with a halogen group and optionally interrupted by -O-, -CO-, -OCO-, or -COO-, which is represented by the substituent Z¹ of the benzene or naphthalene ring as represented by ring A and the substituent Z², may have the halogen group at any position thereof and may be interrupted by -O-, -CO-, -OCO-, or -COO- at any position thereof. That is, the -O-, -CO-, -OCO-, or -COO- may be bonded directly to ring A.
Examples of the alkyl group having 1 to 8 carbon atoms include methyl, ethyl, propyl, isopropyl, butyl, s-butyl, t-butyl, isobutyl, amyl, isoamyl, t-amyl, hexyl, cyclohexyl, heptyl, isoheptyl, t-heptyl, n-octyl, isooctyl, t-octyl, 2-ethylhexyl, chloromethyl, dichloromethyl, trichloromethyl, trifluoromethyl, pentafluoroethyl, methoxy, ethoxy, propoxy, isopropoxy, butoxy, s-butoxy, t-butoxy, isobutoxy, amyloxy, isoamyloxy, t-amyloxy, hexyloxy, cyclohexyloxy, heptyloxy, isoheptyloxy, t-heptyloxy, n-octyloxy, isooctyloxy, t-octyloxy, 2-ethylhexyloxy, chloromethoxy, dichloromethoxy, trichloromethoxy, trifluoromethoxy, pentafluoroethoxy, 2-hydroxyethoxy, 2-methyl-2-hydroxyethoxy, 1-methyl-2-hydroxyethoxy, 3-hydroxypropoxy, 2-(2-hydroxyethoxy)ethoxy, 2-methoxyethoxy, 2-butoxyethoxy, 2-methyl-2-methoxyethoxy, 1-methyl-2-methoxyethoxy, 3-methoxypropoxy, 2-(2-methoxyethoxy)ethoxy, acetyl, acetonyl, butan-2-on-1-yl, butan-3-on-1-yl, cyclohexan-4-on-1-yl, trichloroacetyl, trifluoroacetyl, acetoxy, ethanecarbonyloxy, propanecarbonyloxy, butanecarbonyloxy, and trifluoroacetoxy. Examples of the hydrocarbon group having 1 to 8 carbon atoms possessed by the sulfonyl or the sulfinyl group represented by Z¹ or Z² include an alkyl group, such as methyl, ethyl, propyl, isopropyl, butyl, s-butyl, t-butyl, isobutyl, amyl, isoamyl, t-amyl, hexyl, cyclohexyl,
cyclohexylmethyl, 2-cyclohexylethyl, heptyl, isoheptyl, t-heptyl, n-octyl, isooctyl, t-octyl, or 2-ethylhexyl; an alkenyl group, such as vinyl, 1-methylethen-1-yl, propen-1-yl, propen-2-yl, propen-3-yl, buten-1-yl, buten-2-yl, 2-methylpropen-3-yl, 1,1-dimethylethen-2-yl, or 1,1-dimethylpropen-3-yl; an aryl group, such as phenyl, 2-methylphenyl, 3-methylphenyl, 4-methylphenyl, 4-vinylphenyl, 3-isopropylphenyl, 4-isopropylphenyl, 4-butylphenyl, 4-isobutylphenyl, 4-t-butylphenyl, 2,3-dimethylphenyl, 2,4-dimethylphenyl, 2,5-dimethylphenyl, 2,6-dimethylphenyl, 3,4-dimethylphenyl, or 3,5-dimethylphenyl; and an aralkyl group, such as benzyl, 2-methylbenzyl, 3-methylbenzyl, 4-methylbenzyl, phenethyl, 2-phenylpropan-2-yl, or styryl. Examples of the alkyl group having 1 to 8 carbon atoms possessed by the alkylamino or the dialkylamide group represented by Z¹ include the alkyl groups recited above. Examples of the halogen group represented by Z¹ or Z² include fluoro, chloro, bromo, and iodo.

Examples of the ring structure formed by joining a plurality of Z² substituents in general formulae (I) and (IV) include 5- to 7-membered rings, such as cyclopentane, cyclohexane, cyclopentene, benzene, piperidine, morpholine, lactone, and lactam; and fused rings, such as naphthalene and anthracene.

The organic groups having 1 to 30 carbon atoms represented by R² in general formulae (I) and (IV), except those of general formulae (II) and (III), are not limited and include an alkyl group, such as methyl, ethyl, propyl, isopropyl, butyl, s-butyl, t-butyl, isobutyl, amyl, isoamyl, t-amyl, hexyl, cyclohexyl, cyclohexylmethyl, 2-cyclohexylethyl, heptyl, isoheptyl, t-heptyl, n-octyl, isooctyl, t-octyl, 2-ethylhexyl, nonyl, isononyl, decyl, dodecyl, tridecyl, tetradecyl, pentadecyl, hexadecyl, heptadecyl, or octadecyl; an alkenyl group, such as vinyl, 1-methylethenyl, 2-methylethenyl, propenyl, butenyl, isobutenyl, pentenyl, hexenyl, heptenyl, octenyl, decenyl, pentadecenyl, or 1-phenylpropen-3-yl; an alkylaryl group, such as phenyl, naphthyl, 2-methylphenyl, 3-methylphenyl, 4-methylphenyl, 4-vinylphenyl, 3-isopropylphenyl, 4-isopropylphenyl, 4-butylphenyl, 4-isobutylphenyl, 4-t-butylphenyl, 4-hexylphenyl, 4-cyclohexylphenyl, 4-octylphenyl, 4-(2-ethylhexyl)phenyl, 4-stearylphenyl, 2,3-dimethylphenyl, 2,4-dimethylphenyl, 2,5-dimethylphenyl, 2,6-dimethylphenyl, 3,4-dimethylphenyl, 3,5-dimethylphenyl, 2,4-di-t-butylphenyl, or cyclohexylphenyl; an arylalkyl group, such as benzyl, phenethyl, 2-phenylpropan-2-yl, diphenylmethyl, triphenylmethyl, styryl, or cinnamyl; and the hydrocarbon groups recited above which are interrupted with an ether linkage or a thioether linkage, such as 2-methoxyethyl, 3-methoxypropyl, 4-methoxybutyl, 2-butoxyethyl, methoxyethoxyethyl, methoxyethoxyethoxyethyl, 3-methoxybutyl, 2-phenoxyethyl, 2-methylthioethyl, and 2-phenylthioethyl. The above recited groups may be substituted with the following substituents.

The substituents include alkyl groups, such as methyl, ethyl, propyl, isopropyl, cyclopropyl, butyl, s-butyl, t-butyl, isobutyl, amyl, isoamyl, t-amyl, cyclopentyl, hexyl, 2-hexyl, 3-hexyl, cyclohexyl, bicyclohexyl, 1-methylcyclohexyl, heptyl, 2-heptyl, 3-heptyl, isoheptyl, t-heptyl, n-octyl, isooctyl, t-octyl, 2-ethylhexyl, nonyl, isononyl, and decyl; alkoxy groups, such as methoxy, ethoxy, propoxy, isopropoxy, butoxy, s-butoxy, t-butoxy, isobutoxy, amyloxy, isoamyloxy, t-amyloxy, hexyloxy, cyclohexyloxy, heptyloxy, isoheptyloxy, t-heptyloxy, n-octyloxy, isooctyloxy, t-octyloxy, 2-ethylhexyloxy, nonyloxy, and decyloxy; alkylthio groups, such as methylthio, ethylthio, propylthio, isopropylthio, butylthio, s-butylthio, t-butylthio, isobutylthio, amylthio, isoamylthio, t-amylthio, hexylthio, cyclohexylthio, heptylthio, isoheptylthio, t-heptylthio, n-octylthio, isooctylthio, t-octylthio, and 2-ethylhexylthio; alkenyl groups, such as vinyl, 1-methylethenyl, 2-methylethenyl, 2-propenyl, 1-methyl-3-propenyl, 3-butenyl, 1-methyl-3-butenyl, isobutenyl, 3-pentenyl, 4-hexenyl, cyclohexenyl, bicyclohexenyl, heptenyl, octenyl, decenyl, pentadecenyl, eicosenyl, and tricosenyl; arylalkyl groups, such as benzyl, phenethyl, diphenylmethyl, triphenylmethyl, styryl, and cinnamyl; aryl groups, such as phenyl and naphthyl; aryloxy groups, such as phenoxy and naphthoxy; arylthio groups, such as phenylthio and naphthylthio; heterocyclic groups, such as pyridyl, pyrimidyl, pyridazyl, piperidyl, pyranyl, pyrazolyl, triazyl, pyrrolyl, quinolyl, isoquinolyl, imidazolyl, benzimidazolyl, triazolyl, furyl, furanyl, benzofuranyl, thienyl, thiophenyl, benzothiophenyl, thiadiazolyl, thiazolyl, benzothiazolyl, oxazolyl, benzoxazolyl, isothiazolyl, isoxazolyl, indolyl, 2-pyrrolidinon-1-yl, 2-piperidon-1-yl, 2,4-dioxyimidazolidin-3-yl, and 2,4-dioxyoxazolidin-3-yl; halogen atoms, such as fluorine, chlorine, bromine, and iodine; acyl groups, such as acetyl, 2-chloroacetyl, propionyl, octanoyl, acryloyl, methacryloyl, phenylcarbonyl (benzoyl), phthaloyl, 4-trifluoromethylbenzoyl, pivaloyl, salicyloyl, oxaloyl, stearoyl, methoxycarbonyl, ethoxycarbonyl, t-butoxycarbonyl, n-octadecyloxycarbonyl, and carbamoyl; acyloxy groups, such as acetyloxy and benzoyloxy; an amino group; substituted amino groups, such as ethylamino, dimethylamino, diethylamine, butylamino, cyclopentylamino, 2-ethylhexylamino, dodecylamino, anilino, chlorophenylamino, toluidino, anisidino, N-methyl-anilino, diphenylamino, naphthylamino, 2-pyridylamino, methoxycarbonylamino, phenoxycarbonylamino, acetylamino, benzoylamino, formylamino, pivaloylamino, lauroylamino, carbamoylamino, N,N-dimethylaminocarbonylamino, N,N-diethylaminocarbonylamino, morpholinocarbonylamino, methoxycarbonylamino, ethoxycarbonylamino, t-butoxycarbonylamino, n-octadecyloxycarbonylamino, N-methyl-methoxycarbonylamino, phenoxycarbonylamino, sulfamoylamino, N,N-dimethylaminosulfonylamino, methylsulfonylamino, butylsulfonylamino, and phenylsulfonylamino; a sulfonamide group, a sulfonyl group, a carboxyl group, a cyano group, a sulfo group, a hydroxyl group, a nitro group, a mercapto group, an imide group, a carbamoyl group, and a sulfonamido group. These substituents may further be substituted. The carboxyl group and the sulfo group may form a salt.

Preferred examples of the groups represented by R² except those represented by formulae (II) and (III) are methyl, ethyl, propyl, isopropyl, butyl, s-butyl, and t-butyl.

Examples of the alkyl group having 1 to 10 carbon atoms represented by Y¹ in general formulae (I) and (IV) include methyl, ethyl, propyl, isopropyl, butyl, s-butyl, t-butyl, isobutyl, amyl, isoamyl, t-amyl, hexyl, cyclohexyl, cyclohexylmethyl, cyclohexylethyl, heptyl, isoheptyl, t-heptyl, n-octyl, isooctyl, t-octyl, 2-ethylhexyl, nonyl, isononyl, and decyl. Examples of the aryl group having 6 to 20 carbon atoms represented by Y¹ are phenyl, naphthyl, anthracen-1-yl, and phenanthrene-1-yl. Examples of the arylalkyl group having 7 to 20 carbon atoms represented by Y¹ are benzyl, phenethyl, 2-phenylpropane, diphenylmethyl, triphenylmethyl, styryl, and cinnamyl.

In general formulae (I) and (IV), examples of the anion as represented by An^{q-} which is monovalent include halide anions, such as chloride ion, bromide ion, iodide ion, and fluoride ion; inorganic anions, such as perchlorate ion, chlorate ion, thiocyanate ion, hexafluorophosphate ion, hexafluoroantimonate ion, and tetrafluoroborate anion; organic sulfonate anions, such as benzenesulfonate ion, toluenesulfonate ion, trifluoromethanesulfonate ion, diphenylamine-4-sulfonate ion, 2-amino-4-methyl-5-chlorobenzenesulfonate anion, 2-amino-5-nitrobenzenesulfonate anion, and the sulfonate anion described in JP 2004-53799A; organic phosphate anions, such as octylphosphate anion, dodecylphosphate anion, octadecylphosphate anion, phenylphosphate anion, nonylphosphate anion, and 2,2'-methylenebis(4,6-di-tert-butylphenyl)phosphate anion; bis(trifluoromethylsulfonyl)imide anion, bis(perfluorobutanesulfonyl)imide anion, perfluoro-4-ethylcyclohexanesulfonate anion, and tetrakis(pentafluorophenyl)borate anion. Examples of the anion An^{q-} which is divalent include benzenedisulfonate anion and naphthalenedisulfonate anion. If desired, a quencher anion capable of deexciting (quenching) an active molecule in an excited state, a metallocene compound anion of, for example, a ferrocene or a ruthenocene compound having an anionic group (e.g., a carboxyl group, a phosphonic acid group, or a sulfonic acid group) on its cyclopentadienyl ring can be used.

Examples of the quencher anion include anions represented by general formulae (A) and (B) and formulae (C) and (D) shown below and those described in JP 60-234892A, JP 5-43814A, JP 5-305770A, JP 6-239028A, JP 9-309886A, JP 9-323478A, JP 10-45767A, JP 11-208118A, JP 2000-168237A, JP 2002-201373A, JP 2002-206061A, JP 2005-297407A, JP 7-96334B, and WO98/29257.

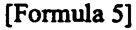

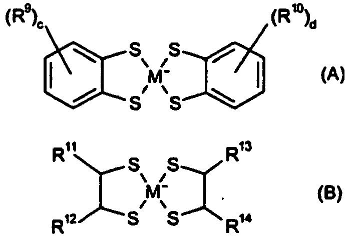

wherein M is as defined for general formula (III); R⁹ and R¹⁰ each independently represent a halogen atom, an alkyl group having 1 to 8 carbon atoms, an aryl group having 6 to 30 carbon atoms, or -SO₂-J; J represents an alkyl group, an optionally halogen-substituted aryl group, a dialkylamino group, a diarylamino group, a piperidino group, or a morpholino group; c and d each independently represent an integer of 0 to 4; and R¹¹, R¹², R¹³, and R¹⁴ each independently represent an alkyl group, an alkylphenyl group, an alkoxyphenyl group, or a halogenated phenyl group.

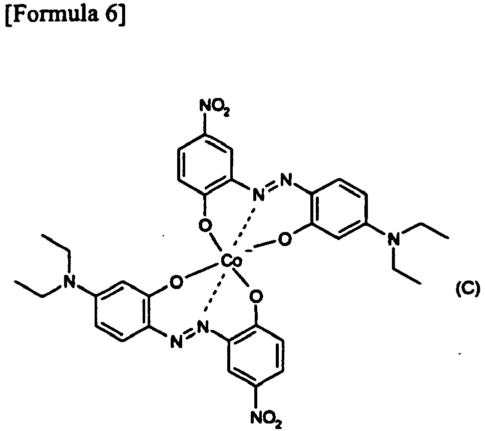

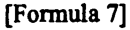

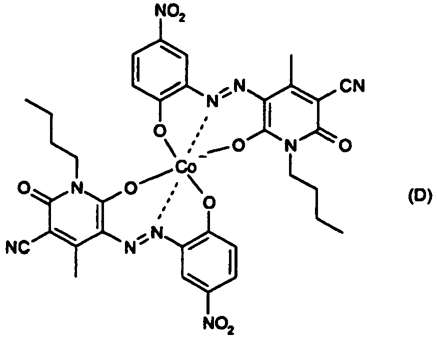

In general formula (II), examples of the optionally halogen-substituted alkyl group having 1 to 4 carbon atoms as represented by R⁰¹, R⁰², R⁰³, and R⁰⁴ include methyl, ethyl, propyl, isopropyl, butyl, s-butyl, t-butyl, and isobutyl. Examples of the optionally halogen-substituted alkoxy group having 1 to 4 carbon atoms include methoxy, ethoxy, propoxy, and isopropoxy. Examples of the halogen atom are fluorine, chlorine, bromine, and iodine. Examples of the cycloalkene ring formed by joining R⁰¹ and R⁰⁴ include cyclobutene ring, cyclopentene ring, cyclohexene ring, and cyclohexadiene ring. Examples of the heterocyclic ring formed by joining R⁰¹ and R⁰⁴ include dihydropyran ring, pyrroline ring, pyrazoline ring, indoline ring, pyrrole ring, thiophene ring, pyridine ring, pyrazine ring, pyrimidine ring, pyridazine ring, triazine ring, quinoline ring, isoquinoline ring, imidazole ring, oxazole ring, imidazolidine ring, pyrazolidine ring, isoxazolidine ring, and isothiazolidine ring. These rings may be fused with other ring(s) or may be substituted.

In general formula (III), examples of the alkyl group having 1 to 4 carbon atoms as represented by R^{a}, R^{b}, R^{c}, R^{d}, R^{e}, R^{f}, R^{g}, R^{h}, and Rⁱ include methyl, ethyl, propyl, isopropyl, butyl, s-butyl, t-butyl, and isobutyl. Examples of the alkyl group a methylene moiety of which is substituted with -O- include methoxy, ethoxy, propoxy, isopropoxy, methoxymethyl, ethoxymethyl, and 2-methoxyethyl. Examples of the alkyl group a methylene moiety of which is substituted with -CO- include acetyl, 1-carbonylethyl, acetylmethyl, 1-carbonylpropyl, 2-oxobutyl, 2-acetylethyl, and 1-carbonylisopropyl.

Examples of the alkylene group having 1 to 8 carbon atoms as represented by Q in general formula (III) include methylene, ethylene, propylene, methylethylene, butylene, 1-methylpropylene, 2-methylpropylene, 1,2-dimethylpropylene, 1,3-dimethylpropylene, 1-methylbutylene, 2-methylbutylene, 3-methylbutylene, 4-methylbutylene, 2,4-dimethylbutylene, 1,3-dimethylbutylene, pentylene, hexylene, heptylene, octylene, ethane-1,1-diyl, and propane-2,2-diyl. Examples of the alkylene group whose methylene moiety is substituted with -O-, -S-, -CO-, -COO-, -OCO-, -SO₂-, -NH-, -CONH-, NHCO-, -N=CH-, or -CH=CH- include methyleneoxy, ethyleneoxy, oxymethylene, thiomethylene, carbonylmethylene, carbonytoxymethylene, methylenecarbonyloxy, sulfonylmethylene, aminomethylene, acetylamino, ethylenecarboxyamide, ethaneimidoyl, ethenylene, and propenylene.
The metal atom as represented by M include Fe, Co, Ni, Ti, Cu, Zn, Zr, Cr, Mo, Os, Mn, Ru, Sn, Pd, Rh, Pt, and Ir.

Of the indolium compounds according to the invention preferred are those represented by general formula (IV) in terms of low production cost and large molar absorptivity.

Preferred examples of the compounds of general formula (I) include compound Nos. 1 through 10 and 12 through 37, whose structural formulae are illustrated below in which only cations are shown. The polymethine chain in the compounds of the invention may take on a resonant structure.

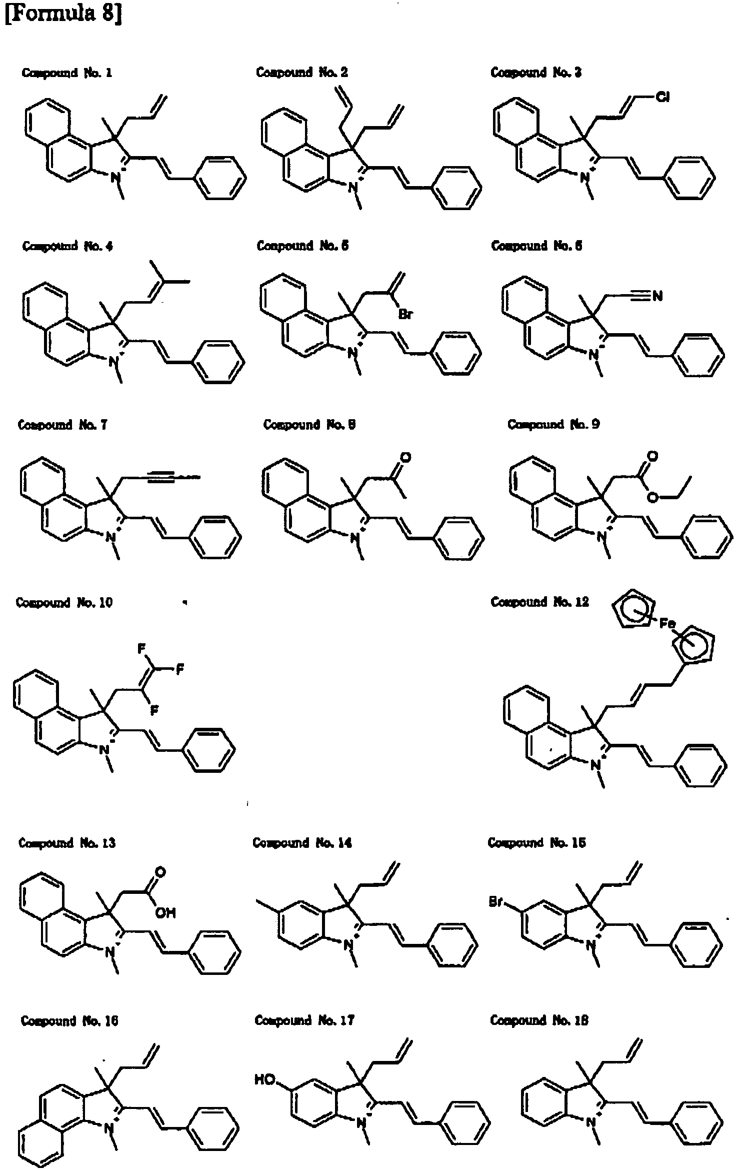

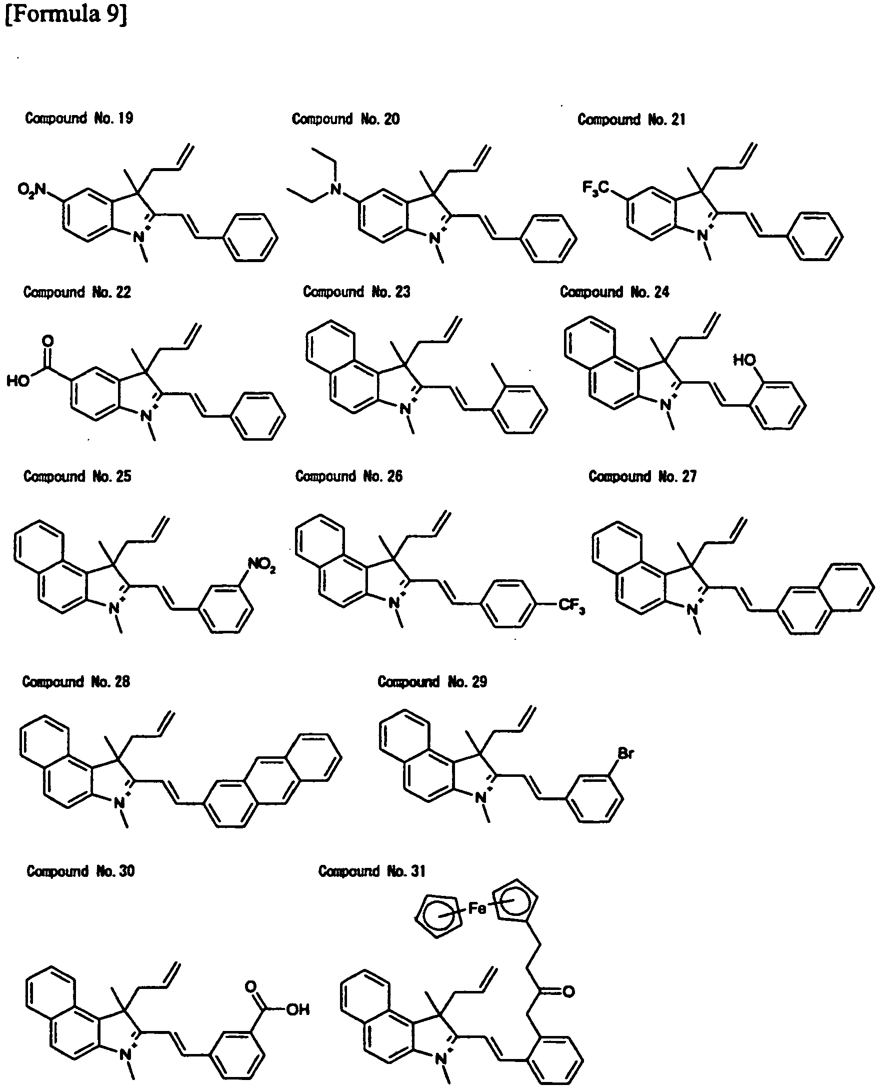

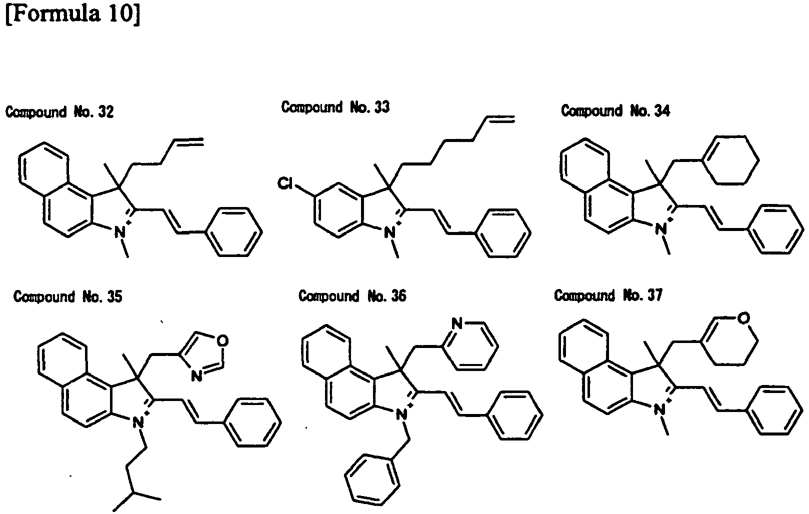

Some of the indolium compounds represented by general formula (I) of the invention embrace optical isomers including enantiomers, diastereomers, and racemates thereof having a chiral center at the asymmetric atom to which R¹ and R² are bonded. Any of these optical isomers, either individual or mixed, is usable.

The indolium compounds of general formula (I) are not restricted by the process of preparation. The indolium compound may be synthesized by, for example, the condensation reaction between a 2-methylindole derivative and an aromatic aldehyde derivative, followed by salt exchange.

The group having a multiple bond represented by general formula (II) can be introduced in the course of preparing a 2-methylindole derivative as an intermediate. For example, an arylhydrazine derivative as a starting material is allowed to react with a 2-butanone derivative having the multiple bond group of general formula (II) to form an indole ring, or a halogenated derivative is allowed to react on an indole ring. Y can be introduced by using Y-D (wherein D is a halogen group, e.g., chlorine, bromine or iodine, or a sulfonyloxy group, e.g., phenylsulfonyloxy, 4-methylphenylsulfonyloxy or 4-chlorophenylsulfonyloxy) reactive with NH of an indole ring. The 2-butanone derivative having the multiple bond group represented by general formula (II) can be obtained by the reaction between acetone and an aldehyde having the multiple bond group.

The indolium compound of the invention is useful not only as an optical recording material but also as an optical element, such as a light absorber used in an optical filter, an intermediate for synthesis of pharmaceuticals, agricultural chemicals, perfumes, dyes, and so forth, or a raw material of various polymers useful as functional materials. Nevertheless, the invention is not limited by these applications.

The optical recording material and the optical recording medium according to the invention will then be described.
The optical recording material of the invention contains at least one of the above described indolium compounds. The optical recording medium of the invention is obtained by forming an optical recording layer of the optical recording material on a substrate.

The method of preparing the optical recording material of the invention and the method of making the optical recording medium of the invention having an optical recording layer of the optical recording material provided on a substrate are not particularly limited. A wet coating technique is generally used to make the optical recording medium, in which an optical recording material in the form of solution is applied to a substrate by spin coating, spraying, dipping or a like method. The optical recording material in the form of solution is prepared by dissolving the indolium compound of the invention and, if necessary, various compounds described later in an organic solvent. Examples of the organic solvent include lower alcohols, such as methanol and ethanol; ether alcohols, such as methyl cellosolve, ethyl cellosolve, butyl cellosolve, and butyl diglycol; ketones, such as acetone, methyl ethyl ketone, methyl isobutyl ketone, cyclohexanone, and diacetone alcohol; esters, such as ethyl acetate, butyl acetate, and methoxyethyl acetate; acrylic esters, such as ethyl acrylate and butyl acrylate; fluoroalcohols, such as 2,2,3,3-tetrafluoropropanol; hydrocarbons, such as benzene, toluene, and xylene; and chlorinated hydrocarbons, such as methylene dichloride, dichloroethane, and chloroform. The formation of the optical recording layer may also be achieved by vapor deposition, sputtering, or a like technique. In the case of using an organic solvent, the amount of the organic solvent is preferably such that the concentration of the indolium compound in the optical recording material of the invention may range from 0.1 % to 10% by mass.

The optical recording layer is formed as a thin film with a thickness usually of from 0.001 to 10 µm, preferably 0.01 to 5 µm.

The content of the indolium compound in the optical recording material of the invention is preferably 10% to 100% by mass based on the solids content. The optical recording layer is preferably formed such that the content of the indolium compound in the optical recording layer ranges from 50% to 100% by mass. Accordingly, it is more preferred for the optical recording material of the invention to contain 50% to 100% by mass of the indolium compound based on the solids content to give the above-recited preferred indolium compound content in the optical recording layer.

The term "solids content of the optical recording material of the invention" refers to the total amount of components other than non-solid components including an organic solvent. The solids content of the optical recording material is preferably 0.01 % to 100% by mass, more preferably 0.1 % to 10% by mass.

Where necessary, the optical recording material of the invention may contain, in addition to the indolium compound, compounds commonly employed in an optical recording layer, such as azo compounds, phthalocyanine compounds, oxonol compounds, squarylium compounds, indole compounds, styryl compounds, porphin compounds, azulenium compounds, croconic methine compounds, pyrylium compounds, thiopyrylium compounds, triarylmethane compounds, diphenylmethane compounds, tetrahydrocholine compounds, indophenol compounds, anthraquinone compounds, naphthoquinone compounds, xanthene compounds, thiazine compounds, acridine compounds, oxazine compounds, spiropyran compounds, fluorene compounds, and rhodamine compounds. The optical recording material may further contain resins, such as polyethylene, polyester, polystyrene, and polycarbonate; surfactants, antistatic agents, lubricants, flame retardants, radical scavengers (e.g., hindered amines), pit formation accelerators (e.g., ferrocene derivatives), dispersants, antioxidants, crosslinking agents, light resistance imparting agents, and so on. The optical recording material may furthermore contain an aromatic nitroso compound, an aminium compound, an iminium compound, a bisiminium compound, a transition metal chelate compound, and the like as a quencher, e.g., for singlet oxygen. The content of these various compounds in the optical recording material is up to 50% by mass based on the solids content of the optical recording material.

The optical recording material of the invention may contain a diimmonium compound. Incorporation of a diimmonium compound is effective in preventing the resulting optical recording medium from reducing the absorbance retention with time. The amount of the diimmonium compound, if added, is preferably up to 99%, more preferably 50% to 95%, by mass based on the solids content of the optical recording material of the invention.

The substrate on which the optical recording layer is provided may be of any material as long as it is substantially transparent to a write/read (recording/reproducing) light beam, including resins, such as polymethyl methacrylate, polyethylene terephthalate, and polycarbonate, and glass. The substrate may have any shape according to use, including a tape, a drum, a belt, and a disk.

A reflective layer of gold, silver, aluminum, copper, etc. may be formed on the optical recording layer by vacuum evaporation or sputtering. A protective layer may be formed using, for example, an acrylic resin or a UV curing resin.

The optical recording material of the invention is suitable to form an optical recording layer of optical recording media that employ a semiconductor laser for writing and reading, especially, known high-speed single-layer, dual-layer, or multi-layer optical discs, such as CD-Rs, DVD±Rs, HD-DVD-Rs, and BD-Rs.

### EXAMPLES

The present invention will now be illustrated in greater detail with reference to Examples, Comparative Example, and Evaluation Examples, but it should be understood that the invention is not construed as being limited thereto.
Preparation Examples 1 and 2 describe preparation of compound No. 1 of general formula (I) in the form of bromide salt and in the form of quencher (C) salt. Examples 1 and 2 illustrate production of optical recording materials and optical recording medium Nos. 1 and 2 using the compounds No. 1 obtained in Preparation Examples 1 and 2.

Comparative Example 1 shows the production of a comparative optical recording material and a comparative optical recording medium No. 1 using an indolium compound having a different structure from that of the indolium compound (I).

In Evaluation Example 1 and Comparative Evaluation Example 1, the optical recording medium No. 1 obtained in Example 1 and the comparative optical recording medium No. 1 obtained in Comparative Example 1 were evaluated for heat resistance by determining the absorbance retention at the absorption maximum wavelength (λₘₐₓ) of the UV absorption spectrum. The results obtained are shown in Table 5 below.

### Preparation Examples 1 and 2 - Preparation of Compound No. 1 in Bromide Form and Compound No. 1 in Quencher (C) Salt Form

Compound No. 1 in Bromide Form and Compound No.1 in Quencher (C) Salt Form were synthesized in accordance with the methods described below. The resulting compounds were identified by IR and ¹H-NMR analyses. Table 1 shows the yields and characteristic values (absorption characteristics as a solution (λₘₐₓ and ε at λₘₐₓ) and decomposition temperature) of the resulting compounds. Tables 2 and 3 furnish the identification data. The decomposition temperature (decomp. temp.) shown in Table 1 is a mass loss onset temperature in differential thermal analysis at a rate of temperature rise of 10°C/min.

### Preparation Example 1 - Preparation of Compound No. 1 in Bromide Form

A reactor was charged with 24 mmol of indolenine quaternary salt (bromide), 30 mmol of benzaldehyde, and 38 g of chloroform, and the mixture was stirred at 80°C for 7 hours. The solvent was removed by evaporation. The residue was recrystallized from 20 ml of chloroform to give compound No. 1 in bromide form.

### Preparation Example 2 - Preparation of Compound No. 1 in Quencher (C) Salt Form

A reactor was charged with 0.69 mmol of compound No. 1 in Br salt form, 0.69 mmol of a triethylamine salt of the anion represented by chemical formula (C) described *supra*, and 3.6 g of pyridine, and the mixture was stirred at 60°C for 2 hours. After addition of 8 g of methanol, the reaction mixture was cooled to room temperature. The solid precipitated was collected by filtration and dried under reduced pressure to yield compound No. 1 in quencher anion (C) salt form.

**Table 1**

| Prepn. Example No. | Indolium Compound | | Yield (%) | λₘₐₓ (%) | ε (x10⁴) | Decomp. Temp. (°C) |
|---|---|---|---|---|---|---|
| | Cation | Anion | | | | |
| 1 | compound No. 1 | Br⁻ | 14 | 363.0 | 1.25 | 201 |
| 2 | compound No. 1 | quencher (C) | 23 | 543.0 | 7.54 | 238 |

**Table 2**

| Prepn. Example No. | Indolium Compound | | IR Absorption Spectrum (cm⁻¹) |
|---|---|---|---|
| | Cation | Anion | |
| 1 | compound No. 1 | Br⁻ | 3420, 2976, 2927, 1609, 1593, 1573, 1521, 1483, 1450, 1396, 1354, 1287, 1247 |
| 2 | compound No. 1 | quencher (C) | 3423, 2975, 1610, 1579, 1519, 1457, 1391, 1340, 1321, 1286, 1263, 1170, 1141 |

**Table 3**

| Prepn. Example No. | Indolium Compound | | ¹H-NMR (DMSO-d6) |
|---|---|---|---|
| | Cation | Anion | |
| 1 | compound No. 1 | Br⁻ | 8.54 (d, 1H), 8.48 (d, 1H), 8.30 (d, 1H), 8.23-8.21 (m, 3H), 8.10 (d, 1H), 7.83-7.58 (m, 6H), 4.88 (m, 1H), 4.73-4.60 (m, 2H), 4.32 (s, 3H), 3.62-3.49 (m, 2H), 2.05 (s, 3H) |
| 2 | compound No. 1 | quencher (C) | 8.99 (d, 2H), 8.55 (d, 1H), 8.49 (d, 1H), 8.30 (d, 1H), 8.23-8.21 (m, 3H), 8.10 (d, 1H), 7.87-7.58 (m, 10H), 6.55 (d, 2H), 6.37-6.34 (m, 2H), 5.73 (d, 2H), 4.87 (quin, 1H), 4.73-4.63 (m, 2H), 4.31 (s, 3 h), 3.59-3.51 (m, 2H), 3.29 (q, 8H), 2.07 (s, 3H), 1.01 (t, 12H) |

### Examples 1 and 2

Each of the compounds obtained in Preparation Examples 1 and 2 was dissolved in 2,2,3,3-tetrafluoropropanol in a concentration of 1.0% by mass to prepare a solution as an optical recording material. A titanium chelate compound T-50 (available from Nippon Soda Co., Ltd.) was applied to a 12 cm diameter polycarbonate disk substrate, followed by hydrolysis to form a primer layer having a thickness of 0.01 µm. The optical recording material was applied onto the primer layer by spin coating to form an optical recording layer having a thickness of 100 nm. The resulting optical recording media were designated as optical recording medium Nos. 1 and 2. The optical recording medium Nos. 1 and 2 were measured for UV absorption spectrum and UV reflection (incidence angle: 5°, on the optical recording layer) spectrum. The results obtained are shown in Table 4.

**Table 4**

| Example No. | Indolium Compound | | λₘₐₓ (nm) | r405 | λₘₐₓ of Reflected Light (nm) / Reflectance (%) |
|---|---|---|---|---|---|
| | Cation | Anion | | | |
| 1 | compound No. 1 | Br⁻ | 378 | 0.94 | 476 (23.5) |
| 2 | compound No. 1 | quencher (C) | 566 | 0.58 | 582 (34.1) |

| | | | | | |
|---|---|---|---|---|---|
| * r405: ratio of molar absorptivity at 405 nm to that at λₘₐₓ | | | | | |

### Comparative Example 1

An optical recording material was prepared in the same manner as in Examples 1 and 2 except to 5 except for using comparative compound No. 1 shown below in place of the indolium compound of the invention. An optical recording medium (designated No. 1) was made using the resulting optical recording material in the same manner as in Examples.

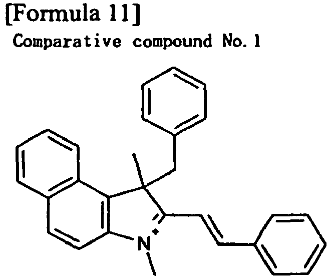

### Evaluation Example 1 and Comparative Evaluation Example 1

Optical recording medium No. 1 obtained in Example 1 and comparative optical recording medium No. 1 obtained in Comparative Example 1 were evaluated for heat resistance. The recording medium was placed in a hot air circulating thermostat dryer set at 80°C for 120 hours. The UV absorption spectra measured before and after the heating were compared to calculate an absorbance retention at the λₘₐₓ of the UV absorption spectrum before the heating. The results obtained are shown in Table 5.

**Table 5**

| | Optical Recording Medium | Indolium Compound | | Retention after 120 hrs (%) |
|---|---|---|---|---|
| | | Cation | Anion | |
| Evaluation Example 1 | optical recording medium No. 1 | compound No. 1 | Br⁻ | 64.6 |
| Comparative Evaluation Example 1 | comparative optical recording medium No. 1 | comparative compound No. 1 | Br⁻ | 0 |

As is apparent from Table 5, the optical recording medium having an optical recording layer formed of the optical recording material of the invention exhibits a high absorbance retention even after being heated at 80°C for 120 hours. The comparative optical recording medium having an optical recording layer formed of the comparative optical recording material containing the comparative compound suffers from a considerable reduction in absorbance retention, proving to have poor heat resistance.

### INDUSTRIAL APPLICABILITY

The present invention provides an indolium compound suited to form an optical recording layer of an optical recording medium and an optical recording material containing the indolium compound. The specific indolium compound according to the invention has a low decomposition temperature and therefore exhibits low heat storage properties and is able to suppress thermal interference. The compound has high light resistance and is suited to form an optical recording layer of an optical recording medium.

## Claims

1. An indolium compound represented by general formula (I): 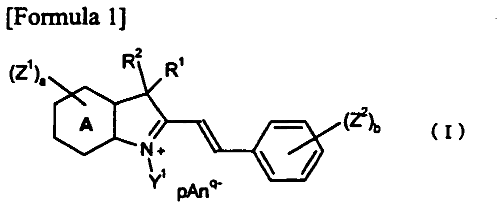 wherein ring A represents a benzene ring or a naphthalene ring, R¹ represents a group represented by general formula (II) or (III); R² represents an organic group having 1 to 30 carbon atoms or a group represented by general formula (II) or (III); Y¹ represents a hydrogen atom, an alkyl group having 1 to 10 carbon atoms, an aryl group having 6 to 20 carbon atoms, or an arylalkyl group having 7 to 20 carbon atoms; Z¹ represents an alkyl group having 1 to 8 carbon atoms optionally substituted with a halogen group and optionally interrupted by -O-, -CO-, -OCO-, or -COO-, a sulfonyl group having a hydrocarbon group having 1 to 8 carbon atoms, a sulfinyl group having a hydrocarbon group having 1 to 8 carbon atoms, an alkylamino group having an alkyl group containing 1 to 8 carbon atoms, a dialkylamino group having an alkyl group containing 1 to 8 carbon atoms, a group represented by general formula (III), a cyano group, a nitro group, a hydroxyl group, or a halogen group; Z² represents an alkyl group having 1 to 8 carbon atoms optionally substituted with a halogen group and optionally interrupted by -O-, -CO-, -OCO-, or -COO-, a sulfonyl group having a hydrocarbon group having 1 to 8 carbon atoms, a sulfinyl group having a hydrocarbon group having 1 to 8 carbon atoms, a group represented by general formula (III), a cyano group, a nitro group, a hydroxyl group, or a halogen group; a plurality of Z² substituents may be joined to form a ring structure; a represents an integer of 0 to 6; b represents an integer of 0 to 5; An^{q-}represents a q-valent anion; q represents 1 or 2; and p represents a number necessary to neutralize an electric charge, 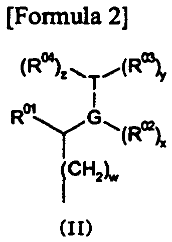 wherein the bond between G and T is a double bond, a conjugated double bond, or a triple bond; G represents a carbon atom; T represents a carbon atom, an oxygen atom, or a nitrogen atom; x, y, and z each represent 0 or 1, provided that, when T is oxygen, y=z=0, and, when T is nitrogen, y+z=0 or 1; w represents an integer of 0 to 4; R⁰¹, R⁰², R⁰³, and R⁰⁴ each independently represent a hydrogen atom, a hydroxyl group, a nitro group, a cyano group, a halogen atom, an alkyl group having 1 to 4 carbon atoms optionally substituted with a halogen atom, or an alkoxy group having 1 to 4 carbon atoms optionally substituted with a halogen atom; R⁰¹ and R⁰⁴ may be joined to form a cycloalkene ring or a heterocyclic ring, 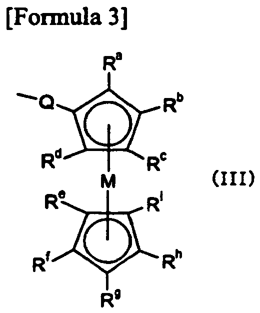 wherein R^{a}, R^{b}, R^{c}, R^{d}, R^{e}, R^{f} R^{g}, R^{h}, and Rⁱ each independently represent a hydrogen atom or an alkyl group having 1 to 4 carbon atoms a methylene moiety of which may be
displaced by -O- or -CO-; Q represents a single bond or an optionally substituted alkylene group having 1 to 8 carbon atoms a methylene moiety of which may be displaced by -O-, -S-, -CO-, -COO-, -OCO-, -SO₂-, -NH-, -CONH-, -NHCO-, -N=CH-, or -CH=CH-; and M represents Fe, Co, Ni, Ti, Cu, Zn, Zr, Cr, Mo, Os, Mn, Ru, Sn, Pd, Rh, Pt, or Ir.

2. The indolium compound according to claim 1, represented by general formula (IV): 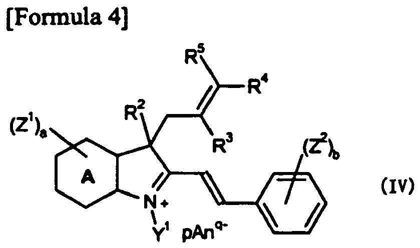 wherein ring A, R², Y¹, Z¹, Z², a, b, An^{q-}, p, and q are as defined for general formula (I); and R³, R⁴, and R⁵ each independently represent a halogen atom, a hydroxyl group, a nitro group, a cyano group, an alkyl group having 1 to 4 carbon atoms optionally substituted with a halogen atom, or an alkoxy group having 1 to 4 carbon atoms optionally substituted with a halogen atom.

3. An optical recording material adapted to be used to form an optical recording layer on a substrate to provide an optical recording medium, the optical recording material comprising at least one indolium compound according to claim 1 or 2.

4. An optical recording medium comprising a substrate and an optical recording layer on the substrate, the optical recording layer being formed of the optical recording material according to claim 3.

## Patentansprüche

1. Indolverbindung, dargestellt durch die folgende allgemeine Formel (I): 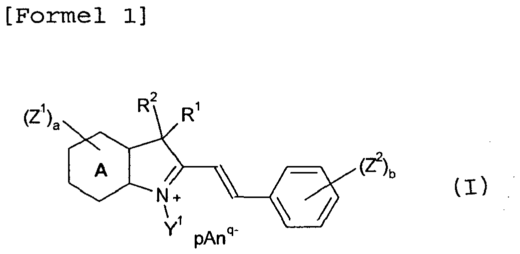 wobei Ring A einen Benzolring oder einen Naphthalinring darstellt; R¹ eine Gruppe darstellt, die durch die folgende allgemeine Formel (II) oder (III) dargestellt ist; R² eine organische Gruppe mit 1 bis 30 Kohlenstoffatomen oder eine Gruppe, die durch die folgende allgemeine Formel (II) oder (III) dargestellt ist, darstellt; Y¹ ein Wasserstoffatom, eine Alkylgruppe mit 1 bis 10 Kohlenstoffatomen, eine Arylgruppe mit 6 bis 20 Kohlenstoffatomen, oder eine Arylalkylgruppe mit 7 bis 20 Kohlenstoffatomen darstellt; Z¹ eine Alkylgruppe mit 1 bis 8 Kohlenstoffatomen, gegebenenfalls substituiert mit einer Halogengruppe und gegebenenfalls unterbrochen durch -O-, -CO-, -OCO-, oder -COO-, eine Sulfonylgruppe, welche eine Kohlenwasserstoffgruppe aufweist, die 1 bis 8 Kohlenstoffatome hat, eine Sulfinylgruppe, welche eine Kohlenwasserstoffgruppe aufweist, die 1 bis 8 Kohlenstoffatome hat, eine Alkylaminogruppe, welche eine Alkylgruppe aufweist, die 1 bis 8 Kohlenstoffatome enthält, eine Dialkylaminogruppe, welche eine Alkylgruppe aufweist, die 1 bis 8 Kohlenstoffatome enthält, eine Gruppe, die durch die allgemeine Formel (III) dargestellt ist, eine Cyanogruppe, eine Nitrogruppe, eine Hydroxylgruppe, oder eine Halogengruppe darstellt; Z² eine Alkylgruppe mit 1 bis 8 Kohlenstoffatomen, gegebenenfalls substituiert mit einer Halogengruppe und gegebenenfalls unterbrochen durch -O-, -CO-, -OCO-, oder -COO-, eine Sulfonylgruppe, welche eine Kohlenwasserstoffgruppe aufweist, die 1 bis 8 Kohlenstoffatome hat, eine Sulfinylgruppe, welche eine Kohlenwasserstoffgruppe aufweist, die 1 bis 8 Kohlenstoffatome hat, eine Gruppe, die durch die allgemeine Formel (III) dargestellt ist, eine Cyanogruppe, eine Nitrogruppe, eine Hydroxylgruppe, oder eine Halogengruppe darstellt; eine Vielzahl von Z² Substituenten miteinander verbunden sein können, um eine Ringstruktur zu bilden; a eine ganze Zahl von 0 bis 6 darstellt; b eine ganze Zahl von 0 bis 5 darstellt; An^{q-} ein q-valentes Anion darstellt; q 1 oder 2 darstellt; und p eine Zahl darstellt, die notwendig ist, um eine elektrische Ladung zu neutralisieren; 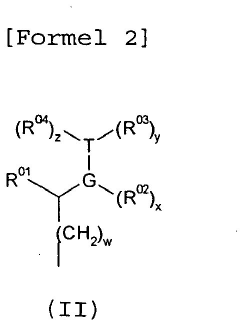 wobei die Bindung zwischen G und T eine Doppelbindung, eine konjugierte Doppelbindung, oder eine Dreifachbindung ist; G ein Kohlenstoffatom darstellt; T ein Kohlenstoffatom, ein Sauerstoffatom, oder ein Stickstoffatom darstellt; x, y und z jeweils 0 oder 1 darstellen; vorausgesetzt, dass, wenn T Sauerstoff ist, y = z = 0, und, wenn T Stickstoff ist, y + z - 0 oder 1; w eine ganze Zahl von 0 bis 4 darstellt; R⁰¹, R⁰², R°³, und R⁰⁴ jeweils unabhängig voneinander ein Wasserstoffatom, eine Hydroxylgruppe, eine Nitrogruppe, eine Cyanogruppe, ein Halogenatom, eine Alkylgruppe mit 1 bis 4 Kohlenstoffatomen, gegebenenfalls substituiert mit einem Halogenatom, oder eine Alkoxygruppe mit 1 bis 4 Kohlenstoffatomen, gegebenenfalls substituiert mit einem Halogenatom, darstellen; R⁰¹ und R⁰⁴ miteinander verbunden sein können, um einen Cycloalkenring oder einen heterocyclischen Ring zu bilden; 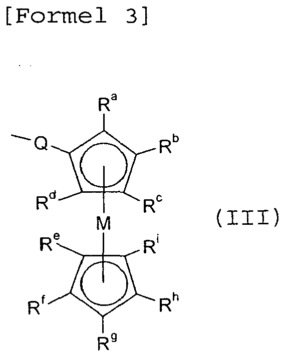 wobei R^{a}, R^{b}, R^{c}, R^{d}, R^{e}, R^{f}, R^{g}, R^{h}, und Rⁱ jeweils unabhängig voneinander ein Wasserstoffatom oder eine Alkylgruppe mit 1 bis 4 Kohlenstoffatomen darstellen, wobei eine Methyleneinheit der Alkylgruppe durch -O- oder -CO- ersetzt sein kann; Q eine Einzelbindung oder eine optional substituierte Alkylengruppe mit 1 bis 8 Kohlenstoffatomen darstellt, wobei eine Methylengruppe der Alkylengruppe durch -O-, -S-, -CO-, -COO-, -OCO-, -SO₂-, -NH-, -CONH-, -NHCO-, -N=CH-, oder -CH=CH- ersetzt sein kann; und M Fe, Co, Ni, Ti, Cu, Zn, Zr, Cr, Mo, Os, Mn, Ru, Sn, Pd, Rh, Pt, oder Ir darstellt.

2. Die Indolverbindung nach Anspruch 1, dargestellt durch die allgemeine Formel (IV): 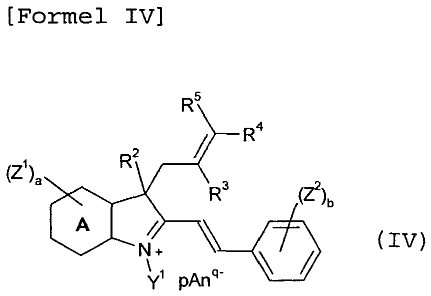 wobei der Ring A, R², Y¹, Z¹, Z², a, b, An^{q-}, p, und q wie für die allgemeine Formel (I) definiert sind; und R³, R⁴, und R⁵ jeweils unabhängig voneinander ein Halogenatom, eine Hydroxylgruppe, eine Nitrogruppe, eine Cyanogruppe, eine Alkylgruppe mit 1 bis 4 Kohlenstoffatomen, die gegebenenfalls mit einem Halogenatom substituiert ist, oder eine Alkoxygruppe mit 1 bis 4 Kohlenstoffatomen, die gegebenenfalls mit einem Halogenatom substituiert ist, darstellen.

3. Optisches Aufzeichnungsmaterial, das eingerichtet ist, um für die Bildung einer optischen Aufzeichnungsschicht auf einem Substrat verwendet zu werden, um ein optisches Aufzeichnungsmedium bereitzustellen, wobei das optische Aufzeichnungsmaterial mindestens eine Indolverbindung gemäß Anspruch 1 oder 2 umfasst.

4. Optisches Aufzeichnungsmedium, umfassend ein Substrat und eine optische Aufzeichnungsschicht auf dem Substrat, wobei die optische Aufzeichnungsschicht aus dem optischen Aufzeichnungsmaterial gemäß Anspruch 3 gebildet wird.

## Revendications

1. Composé d'indolium représenté par la formule générale (I) : 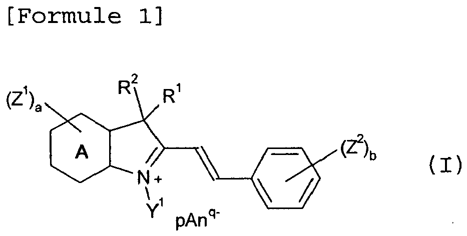 où le cycle A représente un cycle benzénique ou un cycle naphtalénique ; R¹ représente un groupe représenté par la formule générale (II) ou (III) ; R² représente un groupe organique ayant 1 à 30 atomes de carbone ou un groupe représenté par la formule générale (II) ou (III) ; Y¹ représente un atome d'hydrogène, un groupe alkyle ayant 1 à 10 atomes de carbone, un groupe aryle ayant 6 à 20 atomes de carbone, ou un groupe arylalkyle ayant 7 à 20 atomes de carbone ; Z¹ représente un groupe alkyle ayant 1 à 8 atomes de carbone facultativement substitué par un groupe halogène et facultativement interrompu par -O-, -CO-, -OCO-, ou -COO-, un groupe sulfonyle ayant un groupe hydrocarboné ayant 1 à 8 atomes de carbone, un groupe sulfinyle ayant un groupe hydrocarboné ayant 1 à 8 atomes de carbone, un groupe alkylamino ayant un groupe alkyle contenant 1 à 8 atomes de carbone, un groupe dialkylamino ayant un groupe alkyle contenant 1 à 8 atomes de carbone, un groupe représenté par la formule générale (III), un groupe cyano, un groupe nitro, un groupe hydroxyle, ou un groupe halogène, Z² représente un groupe alkyle ayant 1 à 8 atomes de carbone facultativement substitué par un groupe halogène et facultativement interrompu par -O-, -CO-, -OCO-, ou -COO-, un groupe sulfonyle ayant un groupe hydrocarboné ayant 1 à 8 atomes de carbone, un groupe sulfinyle ayant un groupe hydrocarboné ayant 1 à 8 atomes de carbone, un groupe représenté par la formule générale (III), un groupe cyano, un groupe nitro, un groupe hydroxyle, ou un groupe halogène ; une pluralité de substituants Z² peuvent être liés pour former une structure cyclique ; a représente un nombre entier compris entre 0 et 6 ; b représente un nombre entier compris entre 0 et 5 ; An^{q-} représente un anion de valence q ; q représente 1 ou 2 ; et p représente un nombre nécessaire pour neutraliser une charge électrique, 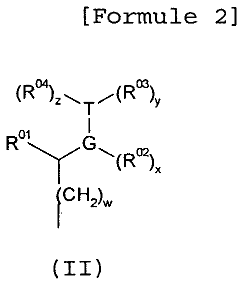 où la liaison entre G et T est une double liaison, une double liaison conjuguée, ou une triple liaison ; G représente un atome de carbone ; T représente un atome de carbone, un atome d'oxygène, ou un atome d'azote ; x, y, et z représentent chacun 0 ou 1, à condition que, lorsque T est un atome d'oxygène, y = z = 0, et, lorsque T est un atome d'azote, y + z = 0 ou 1 ; w représente un nombre entier compris entre 0 et 4 ; R⁰¹, R⁰² R⁰³, et R⁰⁴ représentent chacun indépendamment un atome d'hydrogène, un groupe hydroxyle, un groupe nitro, un groupe cyano, un atome d'halogène, un groupe alkyle ayant 1 à 4 atomes de carbone facultativement substitué par un atome d'halogène, ou un groupe alcoxy ayant 1 à 4 atomes de carbone facultativement substitué par un atome d'halogène ; R⁰¹ et R⁰⁴ peuvent être liés pour former un cycle cycloalcène ou un hétérocycle, 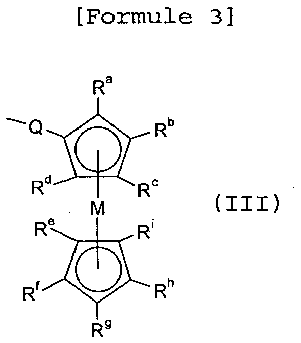 où R^{a}, R^{b}, R^{c}, R^{d}, R^{e}, R^{f}, R^{g}, R^{h}, et Rⁱ représentent chacun indépendamment un atome d'hydrogène ou un groupe alkyle ayant 1 à 4 atomes de carbone, dont un fragment méthylène peut être remplacé par -O- ou -CO- ; Q représente une liaison simple ou un groupe alcylène facultativement substitué ayant 1 à 8 atomes de carbone, dont un fragment méthylène peut être remplacé par -O-, -S-, -CO-, -COO-, -OCO-, -SO₂-, -NH-, -CONH-, -NHCO-, -N=CH-, ou -CH=CH-; et M représente le Fe, Co, Ni, Ti, Cu, Zn, Zr, Cr, Mo, Os, Mn, Ru, Sn, Pd, Rh, Pt, ou le Ir.

2. Composé d'indolium selon la revendication 1, représenté par la formule générale (IV) : 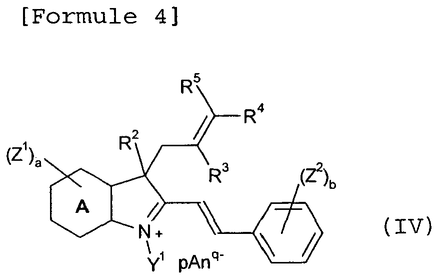 où le cycle A, R², Y¹, Z¹, Z², a, b, An^{q-}, p et q sont tels que définis pour la formule générale (I) ; et R³, R⁴, et R⁵ représentent chacun indépendamment un atome d'halogène, un groupe hydroxyle, un groupe nitro, un groupe cyano, un groupe alkyle ayant 1 à 4 atomes de carbone facultativement substitué par un atome d'halogène, ou un groupe alcoxy ayant 1 à 4 atomes de carbone facultativement substitué par un atome d'halogène.

3. Matériau d'enregistrement optique adapté pour être utilisé pour former une couche d'enregistrement optique sur un substrat pour fournir un support d'enregistrement optique, le matériau d'enregistrement optique comprenant au moins un composé d'indolium selon la revendication 1 ou 2.

4. Support d'enregistrement optique comprenant un substrat et une couche d'enregistrement optique sur le substrat, la couche d'enregistrement optique étant formée du matériau d'enregistrement optique selon la revendication 3.
